# NEW EUROPEAN PATENT SPECIFICATION

(11) **EP 2 026 880 B2**
(45) Date of publication and mention of the opposition decision: **16.04.2025**
(45) Mention of the grant of the patent: 04.07.2018
(21) Application number: 07736621.9
(22) Date of filing: 07.05.2007
(51) Int. Cl.: A61Q 17/00, A61Q 17/04, A61Q 19/00, A61Q 19/08, A61K 8/00, A61K 9/00, A61K 8/25, A61K 33/00, A61P 17/18

(54) **PHARMACEUTICAL AND COSMETIC USE OF SILICA**
PHARMAZEUTISCHE UND KOSMETISCHE VERWENDUNG VON SILIKA
UTILISATION PHARMACEUTIQUE ET COSMÉTIQUE DE SILICE

(30) Priority: 05.05.2006 IS 8442
(43) Date of publication of application: 25.02.2009
(73) Proprietor: Blaa Lonid Hf., 241 Grindavik (IS)
(72) Inventor: EINARSSON, Sigurbjorn, 107 Reykjavik (IS); BRYNJOLFSDOTTIR, Asa, 201 Kopavogur (IS); KRUTMANN, Jean, 41844 Wegberg (DE)
(74) Representative: Arnason Faktor
(86) International application number: PCT/IS2007/000011
(87) International publication number: WO 2007/129330

(56) References cited:
- EP-A1- 1 284 277
- EP-A2- 1 321 432
- EP-A2- 1 640 041
- WO-A1-98/26788
- DE-A1- 102004 014 615
- GB-A- 2 205 239
- US-A- 4 496 357
- US-A- 5 571 518
- US-A1- 2004 151 673
- US-A1- 2006 134 156
- PAILES W H ET AL: "Tripeptide analysis of collagen synthesized by silica-exposed fibroblast cultures", LIFE SCIENCES, PERGAMON PRESS, OXFORD, GB, vol. 32, no. 20, 16 May 1983 (1983-05-16), pages 2379 - 2384, XP025566890, ISSN: 0024-3205, [retrieved on 19830516], DOI: 10.1016/0024-3205(83)90769-5
- SINGH S V ET AL: "The binding of silica to proteins from plasma and lungs of rat: In vitro", CHEMICO-BIOLOGICAL INTERACTIONS, ELSEVIER SCIENCE IRLAND, IR, vol. 49, no. 1-2, 1 April 1984 (1984-04-01), pages 155 - 164, XP025531574, ISSN: 0009-2797, [retrieved on 19840401], DOI: 10.1016/0009-2797(84)90058-9
- "Hunnius Pharmazeutisches Wörterbuch", 2004, Berlin, article HERMANN P T AMMON: "Siliciomdioxid", pages: 1386
- "Auf den Spuren von Fjallagrös und Thorskalysi", PHARMAZEUTISCHE ZEITUNG, 8 November 2003 (2003-11-08), pages 1 - 12
- KARL HECHT, GESUND SEIN UND GESUND WERDEN MIT DER URSUBSTANZ SILIZIUMDIOXID (SI02) SYNONYM KIESELSÄURE, 15 February 2006 (2006-02-15)
- BÄRÖUR SIGURGEIRSSON ET AL.: "The Blue Lagoon and Psoriasis", PATENTINHABERIN BLUE LAGOON, 13 June 2008 (2008-06-13), pages 1 - 12
- Deutsches Ärzteblatt, "KBV ruft nach Erstattungsverbot für Homöopathie" 06.09.2019, [cited 07.04.2021]
- Maibach-Nagel, Egbert, "Homöopathie - Kein Sommerlochthema"Deutsches Ärzteblatt, Vol. 116, 22 July 2019
- Deutsche Welle, "German health insurers urged to end homeopathy refunds" 11.07.2019

## Description

### FIELD OF INVENTION

The present invention is within the field of cosmetics and cosmetically effective ingredients, and specifically concerns the use of silica for cosmetic and/or medical treatment of skin, including improving and enhancing the skin-barrier.

### TECHNICAL BACKGROUND AND PRIOR ART

There is a growing interest in natural ingredient beauty products and products that prevent and improve aging symptoms as well as products that can protect the skin from various environmental stress. A multitude of components from various sources have been used in cosmetic products, although evidence of beneficial effects of individual components is in some cases very limited.

Silica is widely used in cosmetics as a non-active carrier or base component, or a vehicle delivering other components to the skin. Specifically, silica particles are found to adsorb sweat and oil in skin, which prevents light reflection by sweat/oil and keeps make-up on the skin longer. Spherical particle type of silica improves smoothness and spreading of foundation and cream. Fumed silica can be used in liquid formulations to provide rheology control, improved suspension and viscosity stability.

As a recent example, US Patent application No. 2003/0114572 discloses a gel composition that imparts a powdery non-oily feel to the skin. The silica powder in this composition functions as a gelling agent in the composition to form a gel matrix with a non-volatile compound, e.g., squalene, liquid paraffin, C12-C15 alcohol benzoates and the like.

WO 98/26788 suggests the use of silica, as well as other inorganic compounds (zinc oxide and titanium oxide are mentioned) as broad spectrum absorbers or reflectants of radiation but mention the disadvantage that with such compounds the skin assumes a mask-like appearance, i.e. these compounds and formulations are indicated to act more as an added external barrier on top of the skin.

EP 1284277 discloses metal oxide particles having a metal oxide core and a silicon dioxide coating, which may be used in sun screening agents. The silicon dioxide coating is indicated to improve UV-screening properties of the metal oxides by reducing the photocatalytic activity of the metal oxides. Thus, also this prior art reference is directed to an external barrier on the skin.

US 2004151673 discloses a pressurising dispenser comprising a reservoir containing a composition intended for protecting the skin against UV radiation, where the composition comprises (a) a photoprotective system capable of screening out UV radiation (b) generally spherical microparticles of porous silica.

GB 2205239 also discloses an invention that relates generally to infrared blocking and more particularly to a translucent or transparent composition which when applied topically to exposed human skin blocks infrared radiation from the sun and thereby substantially reduces skin damage which results from exposure to unblocked infrared radiation, without creating ludicrous appearance caused by those blockers containing metallic opaquers. A composition is disclosed which contains fumed silica admixed with traditional ultraviolet (UV) absorbers but not opaquers.

Although the mechanisms behind skin aging are not completely understood, the cosmetic market offers an enormous variety of alleged anti-aging products. Most of the products available are directed to the mechanisms of stimulating collagen and glycosaminoglycan synthesis by fibroblasts in the epidermis, lowering free radical levels in the skin or increasing firmness and flexibility of the stratum corneum.

Silica is the general term for silicon dioxide (SiO₂) which is found in nature in several forms and can also be synthesised by precipitating silicious acids and by processes based on acid leaching of silicious minerals. Silica is a major component in bioglass, which is specially designed glass that contains silicon, calcium and sodium oxides. A common formulation has proportions of 45:25:25 % of these components, with the rest being phosphorus oxide. In warm water, such as within the body, sodium at the bioglass surface dissolves. The remaining material is not stable and reorganizes into silica (some of this dissolves) and tiny crystals of hydroxy apatite. The thus obtained porous surface layer is a favourable substrate for the re-growth of bone tissue. Additionally, many such materials appear to stimulate osteoblast turnover and bone formation, as discussed by e.g., by Gao et al. (Silica-based bioacative glasses modulates expression of bone morphogenetic protein-2 mRNA in Sao-2 osteoblasts in vitro. Biomaterials 22: 1475-1483 (2001), Xynos et al. (Bioglass(R)45S5 stimulates osteoblast turnover and enhances bone formation in vitro: Implications and applications for bone tissue engineering. Calcif. Tissue Int. 67: 321-329 (2000), Valerios P., (The effect of ionic products from bioacative glass dissolution on osteoblast proliferation and collagen production. Biomaterials 25:2941-2948 (2004), and Knabe et al. (The effect of bioactive glass ceramics on the expression of bone-related genes and proteins in vitro. Clin. Oral Impl. Res. 16:119-127 (2005), report that a specific tested bioglass material with 60% silica (BG60S) also enhances collagen production in osteoblasts.

Silica has, however, not been indicated as an active agent which itself directly affects physiological processes in the skin that support the natural skin barrier function, induce keratinocyte proliferation, induce collagen synthesis and affords a photo-protective effect.

### Skin barrier enhancement

The skin barrier, also referred to as the epidermal permeability barrier, protects against infection and poisoning, prevents desiccation and is essential for terrestrial life. Barrier function is conferred by the outer layer of epidermis, the stratum corneum which consists of dead, keratin-filled cells embedded in a lipid matrix. Stratum corneum is formed from granular layer keratinocytes during terminal differentiation of normal adult epidermis. As keratinocytes terminally differentiate, they flatten and the intracellular contents are degraded. Lipid-containing lamellar bodies fuse with the plasma membrane and disperse their contents extracellularly. A tough, insoluble cornified envelope is assembled by sequential incorporation of precursor proteins (e.g. involucrin, loricrin and small proline-rich proteins (SPRs)), followed by covalent attachment of extracellular lipid.

Agents that enhance and improve the natural skin barrier function are useful in cosmetic products, e.g. for better retention of moisture in the skin, lessening dry skin formation and for generally keeping the skin in a healthy and visually appealing condition.

### Anti-aging effect by induction of collagen synthesis

Medical research has demonstrated that wrinkle formation occurs due to an imbalance between induction of MMP-1 expression due to photoaging which cannot be compensated by a concomitant induction of collagen synthesis (Scharfetter et al., UVA irradiation induces collagenase in human dermal fibroblasts in vitro and in vivo. Arch. Dermatol. Res. 283: 506-511, 1991). This imbalance is further increased by the fact that UVA irradiation results in a decreased expression of Collagen 1A1 and Collagen 1A2 (Südel et al., Novel Aspects of Intrinsic and Extrinsic Aging of Human Skin: Beneficial Effects of Soy Extract. Photochem. Photobiol. 81: 581-587, 2005). The transcription factor AP1 is activated upon UVA stimulation and exerts the induction of MMP-1 and the repression of Collagen 1A1 and Collagen 1A2 (Chung et al., An AP-1 binding sequence is essential for regulation of the human alpha2(I) collagen (COL1A2) promoter activity by transforming growth factor-beta. J. Biol. Chem. 271:3272-3278, 1996). Hence, agents that stimulate collagen synthesis and preferably as well inhibit photo-reduced up-regulation of MMPs would be very beneficial in cosmetically active skin-care products to provide an anti-aging effect.

### SUMMARY OF INVENTION

The present inventors have found that silica can be used as a cosmetically and pharmaceutically active ingredient. Specifically, as disclosed herein and supported by experimental data, silica exhibits measurable activity in terms of enhancing skin barrier function through enhancing collagen formation.

In one aspect, the invention provides silica for use in the treatment of skin conditions selected from damage due to cortico steroids, skin atrophy, atophic dermatitis, eczema, psoriasis, androsacea.

The invention provides in a further aspect a non-therapeutic cosmetic method for enhancing natural skin barrier function and a non-therapeutic cosmetic method for inducing collagen formation in the skin.

### DETAILED DESCRIPTION OF THE INVENTION

Different types of silica have been tested for the bioactivity underlying the present invention. The preferred silica material according to the invention is natural silica mud precipitated from geothermal saline sources, such as the Blue Lagoon geothermal basin in Iceland ("Blue Lagoon", Svartsengi) with water originating from underground reservoirs filled with very hot geothermal seawater. As the hot underground water reaches the ground surface its temperature and pressure drops leading to silica precipitation, forming a white colloidal mud-like substance with a high salt content, which is referred to herein as "silica mud". The salinity of the geothermal seawater indicates that it is composed of 65% seawater and 35% freshwater. The silica precipitate is collected at about 80°C. The lagoon water has an average temperature of about 37°C, with local and seasonal variations in the range of about 30-45°C, a pH of about 7,5 and a salt content of 2,5 wt%.

For the use in accordance with the present invention, silica mud as described above may be suitably further purified, by removing coarse particles, rinsing and washing. The marine type salts, (mainly NaCL, KCI and CaCl₂) will not be completely rinsed from the material, hence the material will have a significant salt content, which is, however, not considered detrimental and is in fact believed to be advantageous. In certain cosmetic products coarse particles may however be desired and in such cases finer particles can be separated out to give a higher ratio of coarse material (agglomerates).

Even though the above described particular material is found particularly advantageous, the present inventors have conducted as well parallel comparative experiments which clearly indicate that the surprising bioactive effects that have been observed are not limited to silica precipitated from hot saline geothermal sources.

Commercially available colloidal silica has been tested and as is documented herein, such material exhibits significant activity in all the employed activity assays. Consequently, the invention relates as well to the indicated use of silica in any other cosmetically suitable form from other and preferably more conventional sources, e.g. silica from diatoms, refined diatomaceous earth (kieselguhr), colloidal silica in the form a mud-like material or suspension, precipitated silica such as produced from sodium silicate solutions and acid and/or by dissolution of silicate minerals, also various forms of silicic acids may as well be used for the invention, for use as solutions (e.g. supersaturated solutions) or for precipitating silica.

Also, the precipitated geothermal silica mud can be simulated with other forms of silica by admixing the characteristic salts in appropriate amounts to silica in a suitable form.

### Description of silica

The silica which is suitable for use in accordance with the invention will generally have material properties typical for precipitated silica, such as with respect to particle size distribution, specific surface area and porosity. It should be noted that the parameters can be affected by treating silicious material to obtain silica with desired material properties depending on the intended application and formulation. Consequently, in useful embodiments the silica used in the invention will have a BET specific surface are in the range of about 10-200 m²/g, such as in the range of about 10-100 m²/g including the range of 20-100 m²/g or the range of 10-50 m²/g but not limited to those particular ranges, as long as the material exhibits the desired biological properties as exemplified herein. Particle size of silica for use in the invention can vary and the particle size can be affected by treating silica material, e.g. by ultrasound, in order to break up agglomerates into smaller particles. Accordingly silica used as described herein may have a particle size d50 (µm) value in the range from about 2 µm to about 75 µm, such as in the range of 4-50 µm, e.g. in the range of about 4-25 µm or the range of about 10-50 µm. The particle size distribution can be narrow or wide, such as having a d90 value anywhere in the range of about 10-350 µm, where depending on the desired product texture characteristics, the silica can be selected and/or treated to have a generally coarse particle size or a generally fine particle size.

### Skin barrier enhancement

As described above the outer layer of epidermis, the stratum corneum is formed from granular layer keratinocytes during terminal differentiation of normal adult epidermis and as a result, an insoluble cornified envelope is formed by sequential incorporation of precursor proteins including involucrin, loricrin and small proline-rich proteins (SPRs)), followed by covalent attachment of extracellular lipid. It has surprisingly been discovered, as is disclosed in the Examples herein, that silica can enhance the natural skin barrier function, as clearly indicated by experimental data where silica is shown to induce markers for barrier formation in cultured human keratinocytes.

The tested silica mud significantly induced expression of the differentiation markers involucrin, filaggrin and transglutaminase-1, especially at the early time point (24h post addition of the tested substance). The second tested silica sample (sodium metasilicate) showed as well substantial up-regulation.

### Induction of collagen synthesis

The effect of silica on the induction of Col1A1 and Col1A2 expression in human dermal fibroblasts using real time RT-PCR has been verified. The tested silica mud showed a dose dependent expression increase of the collagen genes with a maximal 2-fold expression at 10µg/mL, as described in more detail in the Examples below.

The present invention describes cosmetic and pharmaceutical compositions for enhancing skin barrier function and anti-aging treatment by inducing de novo collagen formation. The compositions are preferably suitably formulated for topical application as described in further detail herein.

In the present context the term "topical application" relates to directly laying on or spreading on outer skin. The term "skin aging" relates to the thinning and/or general degradation of the dermis. This involves a decrease in collagen gene expression, lower fibroblast activity and fibroblast regeneration as well as shrinking of the lamellar barrier, which is shown in the inability of the skin to retain moisture. Accordingly, the term "anti-aging" refers to an effect which counteracts skin aging, i.e., acts against the above effects for retaining smooth and healthy skin.

In an embodiment of the present invention the use of silica is provided for enhancing skin barrier function and/or anti-aging treatment by inducing collagen synthesis, of the skin of a human. As mentioned above, silica useful for the invention is advantageously obtainable from saline hot water environment and can be precipitated from natural saline hot water sources. Suitable silica is however as well obtainable from diatoms and other sources (precipitated silica, silicic acid and the like).

The silica obtained from saline geothermal environment as disclosed herein or another suitable source can be mixed with an acceptable cosmetic carrier to form a cosmetic composition, which can be topically applied to skin. The cosmetic composition can be applied to the skin in cosmetically biologically effective amounts over a period of time sufficient to result in repair or remodelling of the dermis. This repair or remodelling will typically be apparent from a visible improvement of the appearance of the outside of the skin.

The amount of the cosmetic composition, according to the present invention, to be applied to the skin and the duration or number of applications can be determined easily on an individual basis by utilizing the cosmetic composition until a visible improvement of the outer surface of the skin results.

In the present context the term "cosmetic" or "cosmetic composition," according to the invention is intended to include all types of products that are applied in any manner directly to the skin for the purpose of affecting the general visual appearance of the skin. The cosmetic compositions described herein may comprise, in addition to a component of silica disclosed herein, conventional ingredients and carriers used in cosmetic or pharmaceutical products. Said compositions may take the form of a fatty or non-fatty cream, a milky suspension or emulsion of the water-in-oil or oil-in-water type, a lotion, a gel or jelly, a colloidal aqueous or oily solution, a paste, a soap, a foam, an anhydrous base including a powder, an ointment, a semi-soluble tablet (to be suspended in a fluid, such as water) or a stick.

In the present context the term "cream" used herein is meant to encompass all cosmetic cream-type materials which include, for instance, hand creams, cleansing creams, milky lotions, cold creams, vanishing creams, hair creams, foundation creams, beauty washes, facial packs and the like.

The amount of active ingredient contained in the compositions of the present invention may vary between wide limits, depending upon the formulation and the frequency of use of said compositions. Generally, said compositions contain from 0,05%-99% by weight of the silica. However, the preferred amount of the active silica is in the range of about 0,05-10 wt% and more preferably the range of 0,05-5 wt%, including the range of about 0,1-0,5 wt%, such as about 0,10 wt%, 0,15 wt%, 0,20 wt% or about 0,25 wt%. In some products a higher concentration may be useful, e.g. in the range of about 0,25-5 wt% such as in the range of about 0,25-2,5 wt%, including about 0,5 wt% or 1,0 wt%.

The cosmetic compositions used in the method according to the invention can also contain conventional vehicles or carriers, such as solvents, fats, oils and mineral waxes, fatty acids and derivatives thereof, alcohols and derivatives thereof, glycols and derivatives thereof, glycerol and derivatives thereof, sorbitol and derivatives thereof, surface-active agents of the anionic, cationic or non-ionic type, emulsifying agents, preserving agents, perfumes, etc.

The invention also relates to silica for the use in therapeutic treatment of diseases, disorders and conditions which respond to the therapeutic effect disclosed herein. Consequently, these pharmaceutical compositions may be used in the treatment of skin conditions such as skin damage due to cortico steroids, skin atrophy, atophic dermatitis, eczema, psoriasis, rosacea and other skin diseases.

The cosmetic effect of silica for enhancing skin barrier function and anti-aging (collagen synthesis) can be measured by monitoring several biomarkers such as:
- induction of involucrin, transglutaminase-1, filaggrin and loricrin in human keratinocytes.

The monitoring of the biomarkers can be monitored by following the gene expression of these markers using methods such as, but not limited to microarrays and RT-PCR. The monitoring of the biomarkers can further be monitored by measuring protein levels using methods such as, but not limited to immunoassay including ELISA, RIA, EIA and FACS analysis etc., or mass spectrometry including protein microarrays and immunohistochemisty methods.

In another aspect the invention provides a method for enhancing skin barrier function of the skin of a mammal comprising applying to said skin a cosmetic composition with a skin barrier function enhancing amount of silica. The method is set forth as a cosmetic method, i.e. a method for affecting the visual appearance of the skin. The cosmetic composition is suitably selected from any of the conventional types of topical cosmetic formulations such as those described herein above.

As is understood from the description set forth above, the effective amount of silica for inducing collagen formation in the skin can be assessed with methods described in the Examples disclosed.

### BRIEF DESCRIPTION OF FIGURES

FIG. 1 shows results from Example 2, illustrating the inductive effect of the extract of precipitated silica mud at varying concentrations on gene expression of human epidermal keratinocytes.
FIG. 2 shows results from Example 2, illustrating the inductive effect of the extract of sodium metasilicate at varying concentrations on gene expression of human epidermal keratinocytes.
FIG. 4 shows results from Example 3, with different concentration of silica mud and sodium metasilicate on UVA-induced IL-6 gene expression in fibroblasts.
FIG. 5 shows results from Example 4, with different concentration of silica mud and sodium metasilicate on collagen 1A1 and collagen 1A2 gene expression in fibroblasts.

### EXAMPLES

### Example 1: Characterisation of silica used in experiments.

Sample 1: precipitated silica mud from Svartsengi geothermal basin, dried without washing. Sample 2: same source material, washed with water until conductance was below 500 µS/cm. Sample 3: wet silica filter cake (same source as samples 1 and 2).

*Specific surface area and porosity*: adsorption and desorption isotherm for nitrogen at liquid nitrogen temperature were measured with Tristar 3000 from Micromeritics. Surface area and porosity are calculated with software provided with the instrument. Samples were ground and sieved prior to analysis.

*Particle size distribution*: Particle size was determined with Malvern Mastersizer 2000. The samples were suspended in distilled water and the concentration adjusted to the appropriate range for the instrument. In the analysis of the data it is assumed that refraction index for the particles is 1,45 and the adsorption 0,1.

### Results:

**Table 1: Surface characteristics**

| | Sample 1 | Sample 2 |
|---|---|---|
| BET specific surface area (m²/g) | 66,3 | 17,9 |
| t-plot micropore area (m²/g) | 10,9 | 3,0 |
| BJH adsorption cumulative volume of pores between 13 Å and 3000 Å width (cm³/g) | 0,371 | 0,098 |
| BJH adsorption avg. pore width (Å) | 230 | 225 |

**Table 2: Particle size distribution**

| | Sample 3, No pre-treatment | Sample 3 Mixed with water in mech. stirrer for 10 min. | Sample 3 after 2x1 min ultrasonic treatment |
|---|---|---|---|
| d₁₀ (µm) | 10,0 | 5,7 | 0,84 |
| D₅₀ (µm) | 37,3 | 18,9 | 3,97 |
| D₉₀ (µm) | 155 | 42,1 | 10,6 |

The values of the parameters d10, d50 and d90 indicate that 10%, 50% and 90%, respectively, of the total volume of the particles is made up of particles with a diameter smaller than the given value.

### Example 2: Induction of Differentiation in Human Epidermal Keratinocytes bv silica preparates.

The effect of silica mud and sodium metasilicate on the differentiation markers Involucrin, transglutaminase-1, Filaggrin, and Loricrin in human epidermal keratinocytes has been assessed.

### Material & Methods

Silica mud precipitate was obtained from a natural source (Blue Lagoon geothermal basin, Svartsengi, Iceland). Sodium metasilicate was obtained from Prof. Dr. G. Lehmann, Biochemistry, Regensburg University. As control a ceramide mix (10µM) was used to induce the differentiation markers.

Long term cultured normal human epidermal keratinocytes, NHEK, prepared from neonatal foreskin are cultured in Keratinocyte SFM (Invitrogen , Heidelberg, Germany) supplemented with bovine pituitary extract (Invitrogen, Heidelberg, Germany) and recombinant epidermal growth factor (Invitrogen, Heidelberg, Germany). Cells are propagated up to passage 2 or 3 at 37°C and 5% CO₂. For induction of differentiation NHEKs are seeded in 6-well plates and grown up to confluence and treated with several concentrations of the silica preparates.

Total RNA was isolated using RNeasy Total RNA Kits (Qiagen, Hilden, Germany). The RNA concentration was determined via photometric measurement at 260/280 (Biophotometer, Eppendorf AG, Hamburg, Germany). An aliquot of 100ng RNA was used for cDNA synthesis. Superscript^{™}III First-Strand synthesis system for RT-PCR (Invitrogen, Karlsruhe, Germany) was used for the reverse transcription step with random hexamers. For each gene, a specific primer pair was designed by Primer Express^{™} 2.0 software (Applied Biosystems, Darmstadt, Germany) based on the cDNA sequence published as indicated. The following primer pairs were used:

**Table 3: Genes and primer pairs used for real time RT-PCR to determine gene expression in Example 1**

| Gene | Primer pairs | |
|---|---|---|
| 18S rRNA | 5'-GCCGCTAGAGGTGAAATTCTTG-3' | SEQ ID NO: 1 |
| | 5' -CATTCTTGGCAAATGCTTTCG' -3' | SEQ ID NO: 2 |
| Transglutaminase-1 | 5'-CCCCCGCAATGAGATCTACA-3' | SEQ ID NO: 3 |
| | 5' -ATCCTCATGGTCCACGTACACA-3' | SEQ ID NO: 4 |
| Involucrin | 5'-CCCATCAGGAGCAAATGAAAC-3' | SEQ ID NO: 5 |
| | 5'-GCTCGACAGGCACCTTCTG-3 ' | SEQ ID NO: 6 |
| Filaggrin | 5' -AAGGAACTTCTGGAAAAGGAATTTC-3 ' | SEQ ID NO: 7 |
| | 5'-TTGTGGTCTATATCCAAGTGATCCAT-3' | SEQ ID NO: 8 |
| Loricrin | 5' -TCACATTGCCAGCATCTTCTCT-3' | SEQ ID NO: 9 |
| | 5'-GGCTGCTTTTTCTGATAAGACATCT-3' | SEQ ID NO: 10 |

PCR reactions were carried out on an Opticon 1(MJ Research, Waltham, MA, USA) using SYBR Green^{®} PCR Master Mix (Applied Biosystems, Darmstadt, Germany). Each sample was analyzed in double employing the universal protocol over 36 cycles. In detail, 10 minutes 94°C activation of hot start taq polymerase, 20 seconds 95°C denaturation, 20 seconds 55°C annealing, 30 seconds 72°C extension. For comparison of relative expression in real time PCR control cells and treated cells the 2^{(-delta delta C(T))} method was used.

### Results:

As a positive of the art reference a ceramide mix was used which induced the markers transglutaminase-1, involucrin, filaggrin and loricrin to a similar extent (not shown).

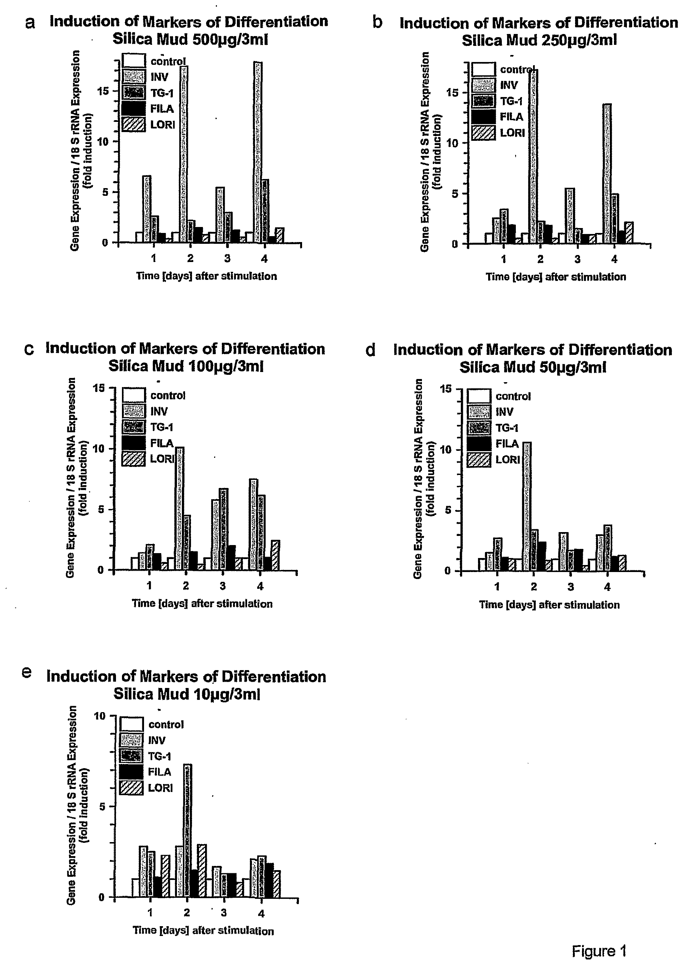

FIG. 1 shows the expression pattern of transglutaminase-1, involucrin, filaggrin and loricrin, in response to silica mud and metasilicate, which all represent skin barrier enhancement.
A rapid aggregation of the keratin cytoskeleton, which causes a collapse of the granular cells into flattened anuclear squames, is a key step in formation of the outermost barrier layer of the skin. This condensed cytoskeleton is cross linked by transglutaminases during formation of the cornified cell envelope (CE). Transglutaminases are expressed and activated during terminal differentiation of Keratinocytes. The membrane-bound form of the transglutaminase-1 and forms ester bonds between specific glutaminyl residues of human involucrin during formation of the cornified cell envelope enzyme. The CE not only prevents water loss but also impedes the entry of allergens and infectious agents. During the last stage of its terminal differentiation of keratinocytes is the formation of a cross linked envelope. This envelope is made up of membrane and cytosolic proteins cross linked by glutamyl lysine isopeptide bonds. Involucrin, being a keratinocyte protein which appears first in the cytoplasm and later becomes cross linked to membrane proteins by transglutaminase, is the most abundant component is of the envelope. Loricrin is another major component of the cross linked cell envelope of the epidermis of the skin, also known as the cornified cell envelope (CE). Filaggrin is a protein which is expressed in granules in the granular layer of interfollicular epidermis is predominantly composed of the protein profilaggrin. Upon terminal differentiation of granular cells, profilaggrin is proteolytically cleaved into filaggrin peptides. Filaggrin aggregates the keratin cytoskeleton and is therefore a key protein in facilitating epidermal differentiation and maintaining barrier function.

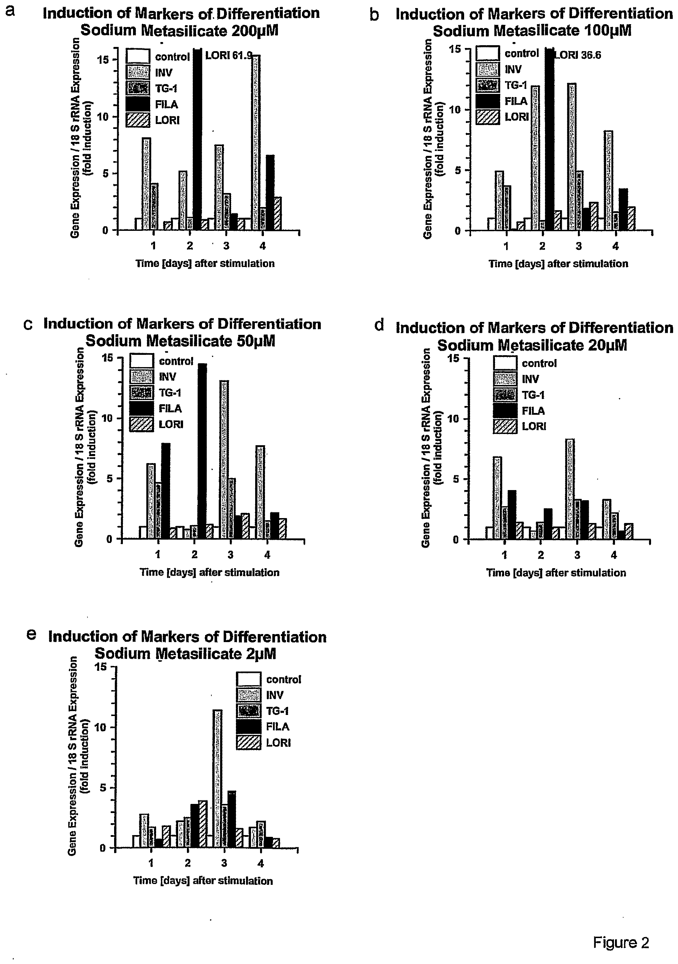

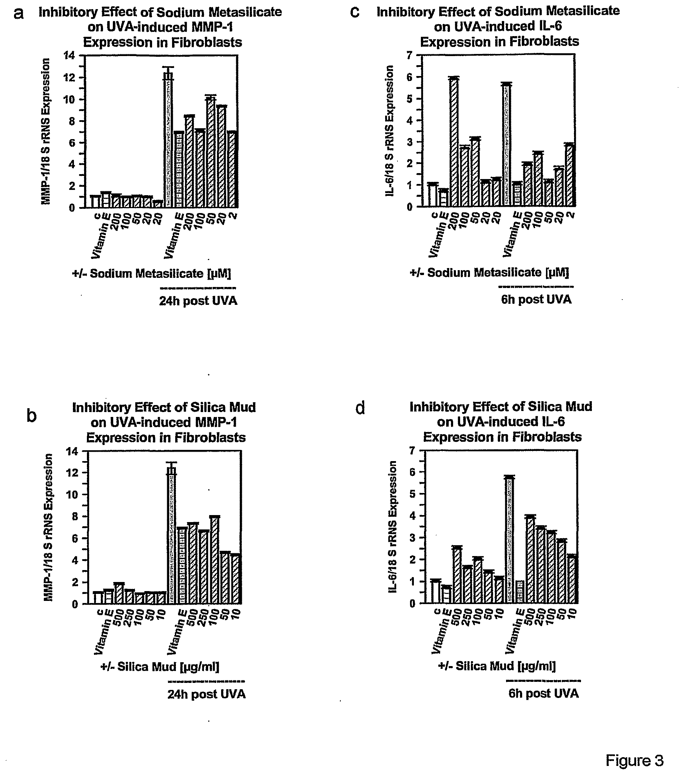

FIG. 1a-e shows induced expression of all four markers in response to silica mud. The two highest concentrations of silica mud show a pronounced increase in expression of INV and TG-1. Increased FILA and LORI expression is also observed, but at lower concentrations. FIG. 2 shows the expression pattern of transglutaminase-1, involucrin, filaggrin and loricrin, in response to sodium metasilicate. All four markers are induced in response to metasilicate, as can be seen in FIG. 2a-e, but here the FILA expression is stronger than with silica mud.

As state of the art reference a ceramide mix was used which induced the markers transglutaminase-1, involucrin, filaggrin and loricrin to a similar extent (not shown).

These results show that silica as exemplified with the silica mud and sodium metasilicate not only induce terminal differentiation of keratinocytes, but induces expression of the most important genes in skin barrier enhancement.

### Example 3: Photoaging inhibitory effect of silica

The photo-protective capacity of silica was assessed in UVA-irradiated human dermal fibroblasts. Induction of MMP-1 is a marker for skin aging. UVA-induced up-regulation of the cytokine IL-6 has previously been shown to be a prerequisite of UVA-induced MMP-1 induction.

Human dermal fibroblasts, HDF, prepared from neonatal foreskin are cultured in DMEM supplemented with 10% FBS in 5% CO₂ for 4 days until they reached confluence as described (Vielhaber *et al.,* 2006). For all studies, only early passage (<12) fibroblasts will be used to avoid changes in their original phenotype during subculture.

The fibroblasts are incubated with different amount of colloidal silica and subjected to UV light. Briefly, for UVA radiation the medium was replaced by phosphate buffered saline, lids were removed and cells were exposed to a dose of 30 J/cm² UVA1 using a UVASUN 24,000 system (Sellas GmbH, Dr. Sellmeier Gevelsberg, Germany). The UVA1 output was determined with a UVAMETER type II (Waldmann, Villingen-Schwenningen, Germany) and found to be approximately 150 mW/cm² UVA1 at a tube to target distance of 30 cm (Grether-Beck *et al.,* 1996; Grether-Beck *et al.,* 2003). Controls without silica are run in parallel. After either 6 or 24 h from UV irradiation, cells are collected and total RNA isolated as described above in Example 2. Quantitative PC is run as described above in Example 2 using specific primer pairs as indicated in Table 4. PCR reactions were carried out as in Example 2.

**Table 4: Genes and primer pairs used for real time RT-PCR to determine gene expression in Example 3**

| Gene | Primer Pairs | |
|---|---|---|
| 18S rRNA | 5'-GCCGCTAGAGGTGAAATTCTTG-3' | SEQ ID NO: 1 |
| | 5'-CATTCTTGGCAAATGCTTTCG'-3' | SEQ ID NO: 2 |
| MMP-1 | 5'-GGGAGATCATCGGGACAACTC-3' | SEQ ID NO: 11 |
| | 5'-GGGCCTGGTTGAAAAGCAT-3' | SEQ ID NO: 12 |
| IL-6 | 5'-AGCCGCCCCACACAGA -3' | SEQ ID NO: 13 |
| | 5'-CCGTCGAGGATGTACCGAAT-3' | SEQ ID NO: 14 |

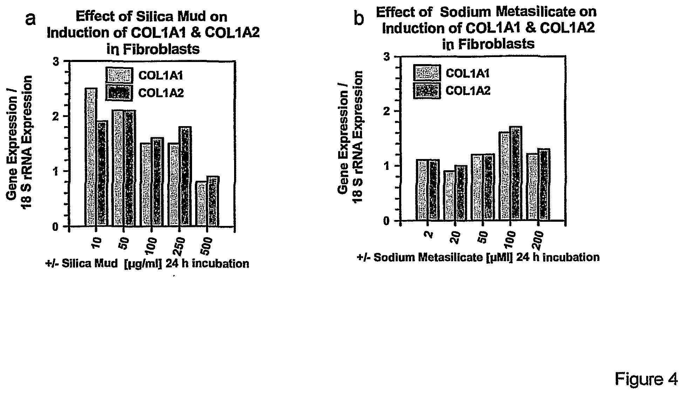

FIG. 4 shows that precipitated Silica mud from geothermal sources inhibits, in a dose dependent manner, the up-regulation of IL-6 and MMP-1. Similar results have been observed for the control substance sodium metasilicate. This strongly indicates that both silica mud (FIG 4a and b) as well as sodium metasilicate (FIG 4c and d) both prevent photoaging of the skin by inhibition of UVA-induced MMP-1 expression in human fibroblasts.

### Example 4: Skin enhancement effect of silica mud and metasilicate

Human dermal fibroblasts, HDF, prepared from neonatal foreskin and cultured for 4 days until they reached confluence as described in Example 3. PCR reactions were carried out as in Example 2 using specific primer pairs as indicated in Table 5.

**Table 5: Genes and primer pairs used for real time RT-PCR to determine gene expression in Example 4**

| Gene | Primer Pairs | Reference |
|---|---|---|
| 18S rRNA | 5'-GCCGCTAGAGGTGAAATTCTTG-3' | SEQ ID NO: 1 |
| | 5'-CATTCTTGGCAAATGCTTTCG'-3' | SEQ ID NO: 2 |
| Collagen 1A1 | 5'-CCTGCGTGTACCCCACTCA-3' | SEQ ID NO: 15 |
| | 5'-ACCAGACATGCCTCTTGTCCTT-3' | SEQ ID NO: 16 |
| Collagen 1A2 | 5'-GATTGAGACCCTTCTTACTCCTGAA-3' | SEQ ID NO: 17 |
| | 5'-GGGTGGCTGAGTCTCAAGTCA-3' | SEQ ID NO: 18 |

FIG. 5 shows the capacity of silica mud to induce the expression of genes coding for the extracellular matrix proteins collagen 1A1 and collagen 1A2, but induced collagen expression prevents skin aging by inducing skin regeneration. Sodium metasilicate did not show any increase in the expression of genes coding for the collagen 1A1 and 1A2. However, the silica mud induced proteins collagen 1A1 and collagen 1A2 expression at a concentration of 10 µg/ml, implicating that silica mud further supports skin regeneration through *de novo* collagen synthesis.

### Example 5: Preparation of skin-care products with biologically active silica for cosmetic or pharmaceutical use

### 5(a) Preparation of a foaming product

**Table 6: Ingredients**

| | | |
|---|---|---|
| A | Water | 80% |
| A | Sodium laureth sulfate | 10,0% |
| A | Sea water (*maris aqua*) | 2,0% |
| A | Cocamidopropyl betaine, cocoglucoside | 3,0% |
| A | Sodium chloride | 1,0% |
| A | Glycerin | 0,80% |
| A | Silica mud | 0,20% |
| B | Phenoxyethanol (and) methylparaben (and) ethylparaben (and) propylparaben (and) butylparaben (and) isobutylparaben | 1,0% |
| B | Preservatives, perfume, minor excipients | 2,0% |

Ingredients of phase A are mixed, ingredients of phase B are mixed, and subsequently phase B is added to and mixed with phase A.

### 5 (b) Preparation of an emulsion product; the following ingredients are mixed:

**Table 7: Ingredients**

| | | |
|---|---|---|
| A | Water | 77% |
| A | Carbomer | 0,4% |
| B | Hydrogenated vegetable glycerides | 2,5% |
| B | Cetearyl alcohol | 2,0% |
| B | C6-C10 Fatty acid triglycerides | 2,0% |
| B | Viscosity adjusting agent(s) | 2,5% |
| B | Further emulsifier agent(s) | 3,5% |
| C | Sea water (*maris aqua*) | 3% |
| C | Glycerin | 1,50% |
| C | Silica mud | 0,20% |
| C | Sodium hydroxide and other pH-adjusters | 0,75% |
| D | Phenoxyethanol (and) methylparaben (and) ethylparaben (and) propylparaben (and) butylparaben (and) isobutylparaben | 1,0% |
| D | Other preservatives, perfume, other minor excipients | 1,6% |
| E | Aluminum starch octenyl succinate | 2,0% |

Ingredients A are mixed at 70°C, ingredients B are mixed at and added to phase A. ingredients C are added in the order shown to the mix at 45°C, ingredients D are added at 40°C and ingredients E are added at 35°C.

### 5 (c) Preparation of powder product; the following ingredients are mixed:

**Table 8: Ingredients**

| | | |
|---|---|---|
| A | Talc | 81,5% |
| B | dimethicone | 1,0% |
| B | kaolin | 9% |
| B | Oriza sativa starch | 2,0% |
| C | Talc | 5,0% |
| C | Silica mud | 0,50% |
| C | preservatives, perfume, anticoagulant, other minor excipients | 1,0% |

Ingredients B are added to A, ingredients C are mixed together and added to the A+B mix.

## Claims

1. Silica for use in the treatment of skin conditions selected from the group consisting of skin damage due to cortico steroids, skin atrophy, atopic dermatitis, eczema, psoriasis, and rosacea.

2. Silica for use according to claim 1, wherein said silica is precipitated from natural saline hot water sources.

3. Silica for use according to claim 1, wherein said silica is obtainable from diatoms.

4. Silica for use according to claim 1, wherein said silica is in a colloidal form.

5. Silica for use according to claim 1, formulated in a form selected from the group consisting of a lotion, a cream, an emulsion including a water-in-oil emulsion and an oil-in-water emulsion, a colloidal solution, a paste, a gel, an ointment, a foam, an anhydrous base, a soap and a suspension.

6. A non-therapeutic cosmetic method for enhancing natural skin barrier function of the skin of a human comprising applying to said skin a cosmetic composition comprising silica as the active ingredient.

7. The method of claim 6, wherein said cosmetic composition is in a form selected from the group consisting of a lotion, a cream, an emulsion including a water-in-oil emulsion and an oil-in-water emulsion, a colloidal solution, a paste, a gel, an ointment, a foam, an anhydrous base, a soap and a suspension.

8. The method of claim 6, where said silica is precipitated from natural saline hot water sources.

9. The method of claim 6, wherein said silica is obtainable from diatoms.

10. The method of claim 6, wherein said silica is in colloidal form.

11. A non-therapeutic cosmetic method for inducing collagen synthesis of the skin of a human comprising applying to said skin a cosmetic composition comprising silica as the active ingredient.

12. The method of claim 11, wherein said cosmetic composition is in a form selected from the group consisting of a lotion, a cream, an emulsion including a water-in-oil emulsion and an oil-in-water emulsion, a colloidal solution, a paste, a gel, an ointment, a foam, an anhydrous base, a soap and a suspension.

13. The method of claim 11, where said silica is precipitated from natural saline hot water sources.

14. The method of claim 11, wherein said silica is obtainable from diatoms.

15. The method of claim 11, wherein said silica is in a colloidal form.

## Patentansprüche

1. Siliciumdioxid zur Verwendung bei der Behandlung von Hautzuständen, die aus der Gruppe ausgewählt sind, welche aus Hautschäden aufgrund von Kortikosteroiden, Hautatrophie, Neurodermitis, Ekzem, Schuppenflechte, und Rosazea besteht.

2. Siliciumdioxid zur Verwendung gemäß Anspruch 1, wobei das Siliciumdioxid aus natürlichen Quellen heißen Salzwassers ausgefällt wird.

3. Siliciumdioxid zur Verwendung gemäß Anspruch 1, wobei das Siliciumdioxid aus Diatomeen gewonnen werden kann.

4. Siliciumdioxid zur Verwendung gemäß Anspruch 1, wobei das Siliciumdioxid in kolloidaler Form vorliegt.

5. Siliciumdioxid zur Verwendung gemäß Anspruch 1, wobei es in einer Form formuliert ist, die aus der Gruppe ausgewählt ist, welche aus einer Lotion, einer Creme, einer Emulsion einschließlich einer Wasser-in-Öl-Emulsion und einer Öl-in-Wasser-Emulsion, einer kolloidalen Lösung, einer Paste, einem Gel, einer Salbe, einem Schaum, einer wasserfreien Base, einer Seife und einer Suspension besteht.

6. Nicht-therapeutisches kosmetisches Verfahren zur Stärkung der natürlichen Hautbarrierewirkung der Haut eines Menschen, wobei es das Aufbringen einer kosmetischen Zusammensetzung, welche Siliciumdioxid als Wirkbestandteil umfasst, auf die Haut umfasst.

7. Verfahren nach Anspruch 6, wobei die kosmetische Zusammensetzung in einer Form vorliegt, die aus der Gruppe ausgewählt ist, welche aus einer Lotion, einer Creme, einer Emulsion einschließlich einer Wasser-in-Öl-Emulsion und einer Öl-in-Wasser-Emulsion, einer kolloidalen Lösung, einer Paste, einem Gel, einer Salbe, einem Schaum, einer wasserfreien Base, einer Seife und einer Suspension besteht.

8. Verfahren nach Anspruch 6, wobei das Siliciumdioxid aus natürlichen Quellen heißen Salzwassers ausgefällt wird.

9. Verfahren nach Anspruch 6, wobei das Siliciumdioxid aus Diatomeen gewonnen werden kann.

10. Verfahren nach Anspruch 6, wobei das Siliciumdioxid in kolloidaler Form vorliegt.

11. Nicht-therapeutisches kosmetisches Verfahren zur Einleitung der Kollagensynthese der Haut eines Menschen, wobei es das Aufbringen einer kosmetischen Zusammensetzung, welche Siliciumdioxid als Wirkbestandteil umfasst, auf die Haut umfasst.

12. Verfahren nach Anspruch 11, wobei die kosmetische Zusammensetzung in einer Form vorliegt, die aus der Gruppe ausgewählt ist, welche aus einer Lotion, einer Creme, einer Emulsion einschließlich einer Wasser-in-Öl-Emulsion und einer Öl-in-Wasser-Emulsion, einer kolloidalen Lösung, einer Paste, einem Gel, einer Salbe, einem Schaum, einer wasserfreien Base, einer Seife und einer Suspension besteht.

13. Verfahren nach Anspruch 11, wobei das Siliciumdioxid aus natürlichen Quellen heißen Salzwassers ausgefällt wird.

14. Verfahren nach Anspruch 11, wobei das Siliciumdioxid aus Diatomeen gewonnen werden kann.

15. Verfahren nach Anspruch 11, wobei das Siliciumdioxid in kolloidaler Form vorliegt.

## Revendications

1. Silice pour utilisation dans le traitement des états pathologiques cutanés choisis dans le groupe constitué par les lésions cutanées dues aux corticoïdes, l'atrophie cutanée, la dermatite atopique, l'eczéma, le psoriasis et la rosacée.

2. Silice pour utilisation selon la revendication 1, où ladite silice est précipitée à partir de sources d'eau chaude saline naturelle.

3. Silice pour utilisation selon la revendication 1, où ladite silice peut être obtenue à partir de diatomées.

4. Silice pour utilisation selon la revendication 1, où ladite silice se présente sous forme colloïdale.

5. Silice pour utilisation selon la revendication 1, formulée sous une forme choisie dans le groupe constitué par une lotion, une crème, une émulsion incluant une émulsion eau dans l'huile et une émulsion huile dans l'eau, une solution colloïdale, une pâte, un gel, un onguent, une mousse, une base anhydre, un savon et une suspension.

6. Méthode cosmétique non thérapeutique pour l'amélioration de la fonction barrière naturelle de la peau d'un humain comprenant l'application à ladite peau d'une composition cosmétique comprenant de la silice au titre de principe actif.

7. Méthode selon la revendication 6, où ladite composition cosmétique se présente sous une forme choisie dans le groupe constitué par une lotion, une crème, une émulsion incluant une émulsion eau dans l'huile et une émulsion huile dans l'eau, une solution colloïdale, une pâte, un gel, un onguent, une mousse, une base anhydre, un savon et une suspension.

8. Méthode selon la revendication 6, où ladite silice est précipitée à partir de sources d'eau chaude saline naturelle.

9. Méthode selon la revendication 6, où ladite silice peut être obtenue à partir de diatomées.

10. Méthode selon la revendication 6, où ladite silice se présente sous forme colloïdale.

11. Méthode cosmétique non thérapeutique pour l'induction de la synthèse de collagène par la peau d'un humain comprenant l'application à ladite peau d'une composition cosmétique comprenant de la silice au titre de principe actif.

12. Méthode selon la revendication 11, où ladite composition cosmétique se présente sous une forme choisie dans le groupe constitué par une lotion, une crème, une émulsion incluant une émulsion eau dans l'huile et une émulsion huile dans l'eau, une solution colloïdale, une pâte, un gel, un onguent, une mousse, une base anhydre, un savon et une suspension.

13. Méthode selon la revendication 11, où ladite silice est précipitée à partir de sources d'eau chaude saline naturelle.

14. Méthode selon la revendication 11, où ladite silice peut être obtenue à partir de diatomées.

15. Méthode selon la revendication 11, où ladite silice se présente sous forme colloïdale.
